(19)

Europäisches
Patentamt
European
Patent Office
Office européen
des brevets

(11) **EP 2 690 516 A1**

(12) **EUROPEAN PATENT APPLICATION**
published in accordance with Art. 153(4) EPC

(43) Date of publication:
**29.01.2014 Bulletin 2014/05**

(21) Application number: **12760992.3**

(22) Date of filing: **19.03.2012**

(51) Int Cl.:
**G05B 19/418** (2006.01)

(86) International application number:
**PCT/JP2012/057012**

(87) International publication number:
**WO 2012/128249 (27.09.2012 Gazette 2012/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.03.2011 JP 2011062574**
**23.03.2011 JP 2011064313**
**23.03.2011 JP 2011064401**
**23.03.2011 JP 2011064436**

(71) Applicant: **NTN Corporation**
**Osaka-shi, Osaka 550-0003 (JP)**

(72) Inventors:
• **YAMAMOTO, Naota**
**Kuwana-shi**
**Mie 511-0811 (JP)**

• **ITO, Takashi**
**Kuana-shi**
**Mie 511-0811 (JP)**
• **KISHIDA, Takashi**
**Kuwana-shi**
**Mie 511-0811 (JP)**
• **KOSAKA, Yoriko**
**Kuwana-shi**
**Mie 511-0811 (JP)**

(74) Representative: **Grünecker, Kinkeldey,**
**Stockmair & Schwanhäusser**
**Leopoldstrasse 4**
**80802 München (DE)**

(54) **METHOD FOR DIAGNOSING REUSABILITY OF MECHANICAL COMPONENT**

(57)  The present invention makes it possible to determine whether a mechanical component can be reused, without consulting a specialist, by: attaching to the mechanical component an IC tag that records identification information that includes at least one among the type, manufacturing time, manufacturing lot, and manufacturing history of the mechanical component, and that enables the identification information to be externally read by an electromagnetic method; recording, in the memory unit of an inspection device that can read the IC tag, inspection items corresponding to the identification information and the evaluation criteria of the inspection items; displaying an inspection item corresponding to the mechanical component on a display unit; obtaining result information from an input unit; and comparing the result information to the evaluation criteria and displaying, on the display unit, whether the part can be reused.

Fig.2

**Description**

TECHNICAL FIELD

This invention relates to a method of determining whether or not a machine element such as a rolling bearing is reusable or calculating the remaining life of the machine element, during maintenance of the machine element.

BACKGROUND ART

Every machine becomes worn and deteriorates with use. In order to use machines for a prolonged period of time, detailed diagnosis and suitable maintenance are indispensable. But since a complicated machine has an extremely large number of machine elements, it is practically difficult to keep track of the production conditions and times for all of these huge number of machine elements so that the individual machine elements can be diagnosed and maintained in optimum manners. Even machine elements of the same kind may have different properties due to different manufacturing conditions, and thus may not be diagnosed under the same standard.

Consideration is now being given to controlling machine elements using IC tags embedded in the individual machine elements. IC tags are memory media into which specific information can be written so that it can be read out. Thus, it is possible to write information on e.g. the properties and conditions of manufacturing steps into an IC tag and read out this  information later.

In the below-identified Patent document 1, a quality control method for machine elements is disclosed in which for each of the component parts forming a machine element, the material, manufacturing steps such as forging and heat treatment, assembling steps and other information are stored in an IC tag, and the IC tag is attached to the corresponding component part so that the information stored in the IC tag can be read out when shipping the machine element or whenever is desired to confirm the material, manufacturing conditions, etc. With this arrangement, since it is possible to keep track of the material and even manufacturing conditions after shipment, it is possible to e.g. find out the cause of any defects.

If an IC tag is attached to a machine element of a machine, such as a rolling bearing, it is possible to write various kinds of identification information such as the type of the machine element, and its production date, production lot and production history. By reading information stored in the IC tag at any time such as during storage, transportation, or before, during or after use, of the machine element, it is possible to instantly confirm its identification information. Thus, by attaching an IC tag to a machine device, it is not necessary to confirm a marking printed on a part at the time of maintenance or failure, in order to find the identification information of the part by referring to a computer record or a printed database kept by e.g. its manufacturer. This improves efficiency of maintenance and repair.

But if an IC tag is simply stuck on the surface of an object, the IC  tag could peel off the object. In order to prevent separation of an IC tag, the below-identified Patent document 2 and other prior art references propose to place the IC tag in a mounting hole formed in a surface of an object and cover the mounting hole with a lid.

The below-identified Patent document 3 discloses a diagnostic method in which the remaining life of a rolling bearing is calculated by analyzing the value measured by an acceleration sensor.

PRIOR ART DOCUMENTS

PATENT DOCUMENTS

Patent document 1: Japanese Patent Publication 2006-53670A
Patent document 2: Japanese Patent Publication 2006-226498A
Patent document 3: Japanese Patent 4504065B

SUMMARY OF THE INVENTION

OBJECT OF THE INVENTION

The method disclosed in Patent document 1 is extremely effective if this method is carried out by the manufacturer of the machine element to be inspected or anyone who has deep knowledge of this particular machine element. But if a machine made up of a large number of machine elements are to be inspected, no experts may be available at least for one of these machine elements. In such a case, for through diagnosis, it is necessary to call in an expert of this particular machine element, or to dispatch this particular machine element to the manufacturer. This prolongs the downtime of the machine, which is not favorable to the user. Especially if the user of the machine is located in a remote area and it is too costly or utterly impossible to ship the machine element, diagnosis itself may be impossible.

The diagnostic method disclosed in Patent document 3 is used exclusively to calculate the remaining life of rolling bearings. It is therefore difficult to use this diagnostic method in calculating the remaining life of a machine element other than rolling bearings because the remaining life has to be calculated in a different manner according to the type of the machine element to be diagnosed. Even when diagnosing a rolling bearing, since its manufacturer and model number are usually not identifiable instantly, and since without this information, it is difficult to diagnose the rolling bearing, it is often necessary to use different means, as explained in claims 2 and 3 of Patent document 3. Thus in order to use the diagnostic method of Patent document 3, it was necessary to keep record of the manufactures and model numbers of all the machine elements to be inspected at one place so that this information is accessible every time a machine element is to be inspected.

An object of the present invention is to provide a diagnostic method which makes it possible to easily and reliably determine whether or not a machine element is reusable, and/or to easily and reliably calculate the remaining life of the machine element, without the need to send the machine element to its manufacturer or to an expert of maintenance and without the need to strictly control machine elements to be diagnosed.

MEANS TO ACHIEVE THE OBJECT

In order to achieve this object, the present invention provides a method of diagnosing a machine element, comprising:

mounting at least one IC tag to the machine element, wherein the IC tag stores identification information on at least one of the kind of the machine element, the time when the machine element was manufactured, a production lot of the machine element, and a production history of the machine element such that the identification information is electromagnetically readable from outside;

preparing an inspection device comprising a communication unit which can read the identification information stored in the IC tag, a memory which stores at least one check item corresponding to the identification information, and at least one of a standard of determination for the check item and a determining equations for calculating a remaining life based on result information for the check item, a display capable of displaying the check item and at least one of a result of determination and the remaining life, an input unit through which the result information for the check item can be entered, and a control unit for determining whether or not the machine element is reusable, thereby obtaining the result of determination, by comparing the result information entered through the input unit with the standard of determination, or calculating the remaining life using the determining equation; and

using the inspection device, calculating the remaining usable time, i.e. the remaining life of the machine element, or determining whether or not the machine element is reusable, after the machine element has been used;

wherein in calculating the remaining life or determining whether or not the machine element is reusable using the inspection device, the identification information of the machine element is read from the IC tag by the communication unit; inspection is carried out based on the check item after the check item has been displayed on the display; the result information, which is a result of carrying out the inspection item, is entered through the input unit; and the remaining life and/or the result of determination is displayed on the display.

In short, the present invention provides the above inspection device, which can calculate the remaining life of a machine element and can determine whether or not the machine element is reusable without the need for an expert, and an environment for this purpose.

This inspection device stores check items corresponding to identification information for each of machine elements of all the types that are to be inspected by the inspection device, and determining equations for calculating the remaining life based on the result information for the check items, and standards of determination. When the IC tag of the machine element is read by the inspection device, at least one check item corresponding to the identification information, i.e. suitable for the particular machine element is displayed on the display. Even a user having no expert knowledge regarding the model number and production lot of the machine element can conduct inspection following the instructions displayed according to the check item. After inspection, the user enters the result information. Based on the entered result information, the inspection device calculates the remaining life of the machine element or determines whether or not the machine element is reusable. The inspection device then displays the result of determination on the display. Thus, it is possible to calculate the remaining life or determine whether or not the machine element is still usable under a uniform standard without the need for difficult determination by an expert.

The above determination may be made using a single determining equation or a plurality of determining equation, or using a composite conditional equation including conditional branches for selecting a plurality of formulas or conditions.

The check item may include, besides items to be inspected on the spot, inputs of a use history. That is, by entering use information of the machine element such as use time, use environment or use conditions, it is possible to calculate the remaining life more reliably by taking into consideration elements that cannot be determined simply by inspection on the spot and at the particular moment.

In a preferred arrangement, the at least one check item comprises a plurality of check items, and the inspection device is configured such that while a first one of the check items is displayed on the display, the result information corresponding to the first one of the check items can be entered, a second one of the check items is displayed on the display after the result information for the first one of the check items has been entered, and after the result information for all of the check items has been entered, determination is made on all of the standards of determination for all of the check items, thereby obtaining the result of determination. With this arrangement, what the user does is simply carry out inspections exactly in the order indicated on the display. Thus anyone can carry out such inspections easily and without error.

When calculating the remaining life too, the inspection device is preferably configured such that while a first one of the check items is displayed on the display, the result information corresponding to the first one of the check items can be entered, a second one of the check items is displayed on the display after the result information for the first one of the check items has been entered, and after the result information for all of the check items has been entered, the determining equation, which contains the result information as variables, is calculated based on the result information for all of the check items. With this arrangement, inspections can be done easily and accurately.

In another preferred arrangement, the at least one check item comprises a plurality of check items, the display is capable of simultaneously displaying the plurality of check items, the inspection device is configured such that the result information corresponding to the plurality of check items can be entered through the input unit, and the determining equation is carried out based on the result information corresponding to the plurality of check items.

When determining whether or not the machine element is reusable, use information including the use time and use conditions of the machine element is preferably used. In particular, preferably, the inspection device is configured such that the above information can be entered, the memory stores a standard of history determination used as a standard in determining whether or not the machine element is reusable, in determining whether or not the machine element is reusable, the control unit also compares the use information with the standard of history determination, thereby obtaining the result of determination. With this arrangement, it is possible to make a determination more properly by also taking into consideration the use history corresponding to the endurance time and use environment, in addition to the inspection on the spot.

Preferably, after calculating the remaining life or determining whether or not the machine element is reusable, the check item(s), use information, remaining life, result of determination, etc. are written into the IC tag so that anyone can see this information later. If the machine element is reused, it is possible to refer to the history of the machine element later. If the machine element is not reused, it is possible to find out the reason why the machine element was determined to be not reusable at any time. Not all but only necessary one or ones of the check items, use information, remaining life and the result of determination may be written into the IC tag.

Preferably, the inspection device includes a battery and is portable. With this arrangement, it is possible to determine whether the machine element is reusable at a location where electric power is not available or not easily accessible, e.g. at a remote uninhabited location.

The inspection device may include a network function such that the inspection device can transmit at least one of the identification information, the use information, the remaining life and the result of determination to a separate machine element control database for storage therein. With this arrangement, by referring to the machine element control database, it is possible to keep track of the status of all of the machine elements, including the information on whether or not the individual machine elements are currently usable or on the current remaining lives of the individual machine elements. Also, it is possible to store any information that cannot be stored in the individual IC tags so that such information can be read out later. It is also possible to calculate the average remaining life of the currently operating machine element, which in turn makes it possible to schedule orders for new machine elements.

If the inspection device has a network function, the inspection device is configured to read the identification information at the communication unit, transmit the identification information thus read to a separate check item control database, and receive, and temporarily store in the memory, the check item corresponding to the identification information, and the standard of determination or the determining equation corresponding to the check item. With this arrangement, without the need to sore the check item, standard of determination and determining equation beforehand, it is possible to receive this information from the database in calculating the remaining life or determining whether or not the machine element is reusable. This allows the inspection device to check a literally infinite number of machine elements, even including machine elements manufactured after the inspection device was manufactured.

If the machine element comprises a plurality of component parts, and if a plurality of IC tags are attached to the respective component parts, the control unit is preferably configured to determine whether or not the machine element is reusable based on whether or not all of the component parts of the machine element satisfy the standard of determination, or based on the result of calculation using the determining equation corresponding to the check item for all of the component parts of the machine element. With this arrangement, it is possible to perform diagnosis or determination in a more detailed and reliable manner.

This arrangement is especially suitable for use in determining reusability or calculating the remaining life, of a bearing. If this method is used for a bearing, IC tags are attached to a plurality of component parts of the bearing such as a seal ring and/or bearing race or races. With this arrangement, it is possible to carry out inspection, calculation and determination that are most suitable for the particular shapes and model numbers of the respective component parts.

When reading the IC tags attached to the respective component parts of the machine element, the machine element may be disassembled into the individual component parts beforehand so that any IC tag that are hidden by another component is exposed and thus readable.

If the machine element includes a portion made of a metal, it is necessary to use an IC tag of the type into which information can be written and from which information can be read even if the IC tag is attached to or embedded in the portion made of a metal.

While numerical values are necessary to calculate the remaining life, the check items for determining whether or not the machine element is reusable does not necessarily need a special-purpose measuring device. For example, it may be possible to determine whether or not the machine element is reusable by visually checking the machine element or striking it with a hammer according to the type of the machine element. This arrangement is favorable especially if there is no specialist and thus no special-purpose measuring device.

ADVANTAGES OF THE INVENTION

According to the present invention, it is possible to calculate the remaining life of a machine element or determine whether or not the machine element is reusable with high reliability at a location where the machine element is being used, without the need to call in a specialist or shipping the machine element to its manufacturer.

BRIEF DESCRIPTION OF THE DRAWINGS

Fig. 1 is a schematic view of a first embodiment of the present invention.

Fig. 2 shows the detailed structure of the first embodiment, illustrating how determination is made on whether or not a machine element is reusable or how the remaining life of the machine element is calculated.

Figs. 3(a), 3(b) and 3(c) show a model number-check item correspondence table, a check item table, and a table of use information to be entered, respectively, which are used when carrying out the diagnostic method according to the present invention.

Fig. 4 shows an example in which a plurality of IC tags are attached to respective component parts of a machine element.

Fig. 5 is a flowchart of a method according to the present invention for determining whether or not the machine element is reusable.

Fig. 6 is a flowchart of a method according to the present invention for calculating the remaining life.

Fig. 7 is flowchart of a method according to the present invention for calculating the remaining life of a rolling bearing in which different formulas are used to calculate the remaining life depending on conditions.

Fig. 8 is a sectional front view of a rolling bearing with an IC tag embodying the present invention, showing an operational state of the bearing.

Fig. 9(a) is a sectional front view of an IC tag mounting structure for the rolling bearing of Fig. 8 according to a first embodiment of the present invention; Fig. 9(b) is a plan view of Fig. 9(a); and Fig. 9(c) is a perspective view of a casing of the mounting structure of Fig. 9(a).

Fig. 10(a) is a sectional front view of an IC tag mounting structure for the rolling bearing of Fig. 8 according to a second embodiment of the present invention; Fig. 10(b) is a plan view of Fig. 10(a); and Fig. 10(c) is a perspective view of a casing of the mounting structure of Fig. 10(a).

Fig. 11 is a sectional front view of a modification of the second embodiment, of which the casing differs from that of the second embodiment in its outer peripheral shape.

Fig. 12 is a sectional front view of an IC tag mounting structure for the rolling bearing of Fig. 8 according to a third embodiment of the present invention.

Fig. 13 is a sectional front view of an IC tag mounting structure for the rolling bearing of Fig. 8 according to a fourth embodiment of the present invention.

Fig. 14(a) is a sectional front view of an IC tag mounting structure for the rolling bearing of Fig. 8 according to a fifth embodiment of the present invention; and Fig. 14(b) is a plan view of Fig. 14(a).

Fig. 15(a) is a perspective view of the casing of the fifth embodiment; and Fig. 15(b) is an exploded perspective view of the casing of Fig. 15(a).

Figs. 16(a) to 16(d) are sectional front views of an IC tag mounting structure according to a sixth embodiment of the invention, showing how the IC tag shown in Fig. 8 is mounted in position by this IC tag mounting structure; and Fig. 16(e) is a perspective view of a lid of the sixth embodiment.

Fig. 17 is a sectional front view of an IC tag mounting structure according to a seventh embodiment of the present invention, showing the state before a mounting surface is ground.

Fig. 18(a) is a sectional front view of an IC tag mounting structure for the rolling bearing of Fig. 8 according to a seventh embodiment of the present invention; and Fig. 18(b) is a plan view of Fig. 18(a).

## BEST MODE FOR EMBODYING THE INVENTION

The embodiments are now described with reference to the drawings.

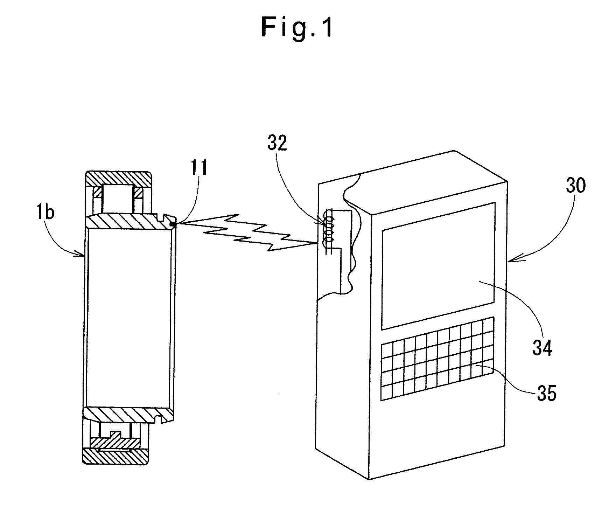

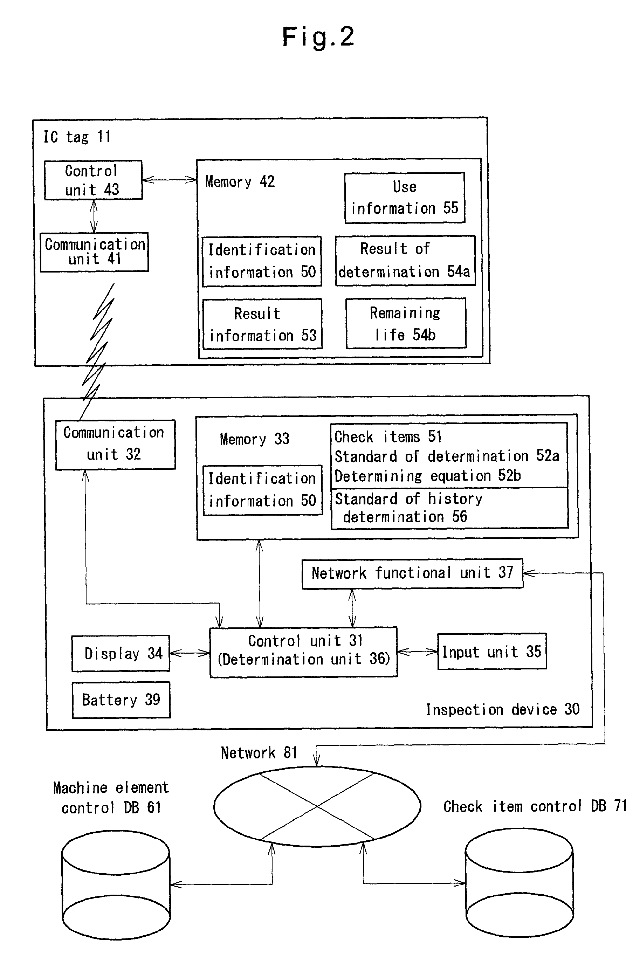

This invention is directed to a diagnostic method including the steps of mounting an IC tag 11 in which identification information on a machine element 1b to the machine element 1b, reading the identification information with an inspection device 30, and diagnosing the machine element 1b. In the diagnosing step, the remaining life of the machine element 1b is calculated by an equation for calculating the remaining life of the machine element and is displayed; and/or determination is made on whether or not the machine element 1b is reusable and the result of determination is displayed. Fig. 1 schematically shows the first embodiment of the diagnostic method according to the present invention. Fig. 2 schematically shows the IC tag 11 and the inspection device 30. The machine element 1b shown is a bearing. The IC tag 11 is attached to a portion of the inner race which is exposed to the outside.

The IC tag 11 is described first. The IC tag 11 is preferably a passive IC tag, which includes a control unit 43 which can read information stored in a memory 42 and can write information into the memory 42 based on electromagnetic induction produced in a communication unit 41 by radio waves from outside. Since this IC tag has to be kept attached to the machine element 1b for a long period of time so that it is possible to read and write information only during maintenance, an active IC tag, which needs a power source, is not suitable as the IC tag 11. Also, to read information easily, the IC tag should be not of the contact type but of the type which can communicate by radio waves at a distance of from several centimeters to about one meter.

When the machine element 1b is manufactured, identification information 50 is stored in the IC tag 11, which includes at least one of the type of the machine element, the time when the machine element was manufactured, its production lot and its production history. The identification information may be stored as raw text data, in the form of codes standardized between the IC tag and the inspection device 30, or as a model number, provided that by referring to the identification information 50, the inspection device 30 can select a suitable check item 51 for the machine element 1b.

The memory 42 of the IC tag 11 has an empty space in which result information 53, result of determination 54a, remaining life 54b, use information 55, etc. can be written.

If the machine element 1b includes a metal portion, the metal portion may interfere with the magnetic flux, making normal reading and writing impossible. Thus, if the machine element 1b has a metal portion and the ID tag 11 is mounted at a position where metal portion could interfere with the magnetic flux from the IC tag, it is necessary to use an IC tag of the type that would not be interfered with by the metal portion even if it is attached to or embedded in the metal portion.

Now the inspection device 30 is described. The inspection device 30 at least includes a communication unit 32, a memory 33, a display 34, an input unit 35, and a control unit 31 for controlling these units.

The communication unit 32 can communicate with the communication unit 41 of the IC tag 11, and includes a coil for producing radio waves and a control circuit for controlling the coil.

The memory 33 is a nonvolatile memory which stores the identification information 50 on the machine element 1b to be diagnosed, check items 51 corresponding to the identification information 50, and a determining equation 52b used to calculate the remaining life based on the standard of determination 52a and the result information 53 of the check items. The control unit 31 reads a check item or items 51 corresponding to the condition of the machine element using the identification information 50 as a key, and also reads the standard of determination 52a regarding the check item or items 51. If necessary, the control unit 31 may read the standard of history determination 56 too.

The check items 51 are stored in the form of sentences or figures, and indicated on the display 34 to inform users of specific check items. This diagnosis, which may include determination on whether the machine element is reusable or calculation of the remaining life, is basically carried on the assumption that the machine element is continuously used after the diagnosis, this diagnosis has to be a nondestructive diagnosis, including visual inspection of the outer appearance of the machine element. What can be confirmed from the inspection of the outer appearance of the machine element by the naked eye or through a magnifying lens includes flaking, seizure, cracks, chipping, breakage, rust, impressions, electrolytic corrosion, and abrasion. There may be check items peculiar to individual machine elements 1b. If, for example, the machine element 1b to be diagnosed is a bearing, check items include items that can be observed from outside, such as breakage of the retainer, smearing, galling, fretting, creeping and flaking. Nondestructive examinations using check devices include confirming the above-mentioned visually observed phenomena by processing photos of the affected areas, measuring dimensions, weight or hardness, assessing condition of rotation, X-ray photography, magnetic particle inspection, fluorescent inspection, and eddy current inspection.

When indicating these check items 51 on the display 34, detailed directions for performing the individual checks are also preferably described and/or shown on the display. For example, if the machine element 1b is a bearing and whether or not the bearing is reusable is to be checked, the following directions may be shown on the display 34: "Search for any flaking on raceways using a magnifying lens with a magnification of 3 or over." If the remaining life is to be determined, the following directions may be shown on the display: "After confirming that no rust is observed on the outer race, measure and enter the weight of the bearing in milligrams." Other check items may include visual checking that needs no special-purpose device, listening to the sound produced when hammering the bearing (only to determine whether the bearing is reusable), measuring the weight or the length, or analysis of the output using a special-purpose device such as an X-ray photographic device or a spectrum analyzer. But preferably, the check items 51 consist of those which can be diagnosed without the need for a special-purpose device so that non-expert users can embody the present invention in an environment close to the site where the machine elements to be diagnosed are being produced.

The standard of determination 52a is the standard in determining whether or not the machine element 1b is reusable based on the results of the diagnosis shown by the check items 51. For example, if the result of the check item 51 can be expressed by either "Yes" or "No", such as "whether or not flaking is visually observed", "whether or not rust is visually observed", "whether or not impressions are observed through a magnifying lens", "whether or not abrasion is observed under a light source" or "whether or not flaking is observed under a scanning electron microscope (SEM)", and if the answer is "No", then it is determined that the machine element is reusable as far as this particular check item goes. For the check item 51 in which a numerical value is measured by e.g. an analyzer or a measuring device, and in which upper and lower limits are set for the measured value, it is determined that the machine element is passable if the measure value is within the range between the upper and lower limits.

The above-mentioned determining equation 52b is a model formula based on which the remaining life can be calculated by entering the result information 53 obtained corresponding to the measured result shown in the check item 51. The optimum content of the determining equation 52b varies not only with the kind of the machine element 1b but also with its manufacturer, or even with the production lot. According to this invention, the inspection device 30 reads an optimum determining equation 52b for the particular machine element 1b indicated by the identification information 50, which is stored in the inspection device 30 or in a database on the network, as well as necessary check items 51.

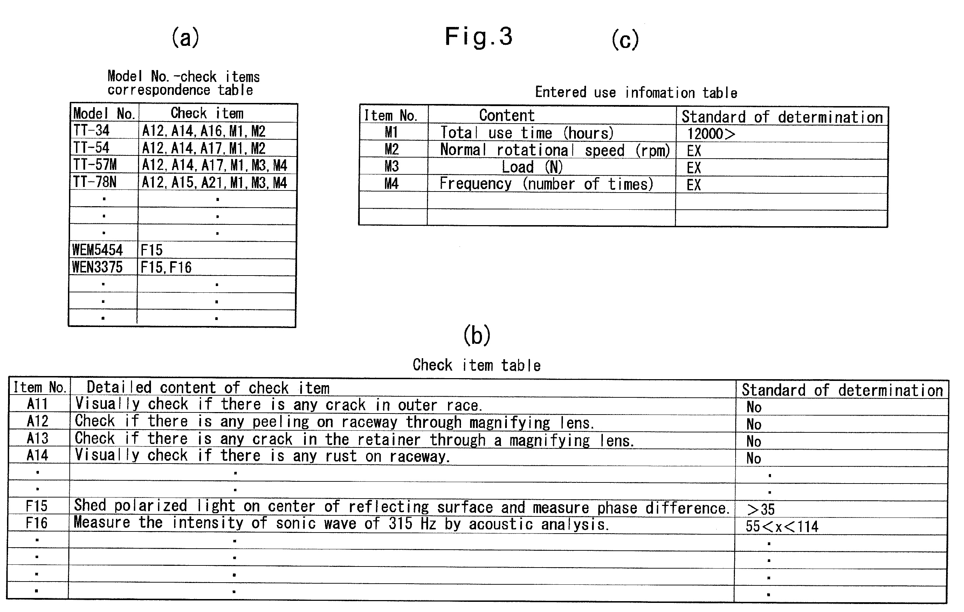

These contents may be stored in the memory 33 in the form of one or more table corresponding to the identification information 50. For the diagnosis on whether or not the machine element is reusable, as shown e.g. in Figs. 3 (a) to 3(c), based on the identification information 50 (which is the model number in this example) received from the IC tag 11, the check items 51 to be carried out are picked up by referring to the model-number-check-item correspondence table, and then the detailed directions and the standards of determination corresponding to the picked-up check items 51 are read out by referring to the check item table.

Standards of history determination 56 are also stored in the memory 33 which are required for the respective check items 51 and entered through the input unit 35, to determine whether or not the machine element is reusable based on the use information 55. The control unit 31 determines whether or not the machine element is reusable when the use information 55 is entered based on the standard of history determination 56. The use information 55 is not directed to the current diagnosis but to specifically how the machine element has been used before the diagnosis, such as the operating time of the machine element 1b, loads applied, rotational speeds and temperatures during operation, and the frequency of uses. The standards of history determination 56 include determining that the machine element is not reusable if the operating time in the use information 55 is longer than a predetermined value, or if the frequency of uses during which loads applied are higher than a predetermined value is larger than a predetermined value. Fig. 3(c) shows examples thereof.

In calculating the remaining life too, necessary information is stored in the memory 33 in the form of one or more tables corresponding to the identification information 50. Based on the identification information 50 (which is the model number in this example) received from the IC tag 11, check items 51 are obtained to obtain a determining equation 52b used and result information 53 (including use information 55) to be entered into the equation 52b. Check items 51 include the description of measuring methods, measuring conditions and measuring devices. But it is not necessary to describe everything. According to the types of users of the inspection device, the above information may be designated by JIS numbers.

The use information 55 is not directed to the current diagnosis but to specifically how the machine element has been used before the diagnosis, such as the operating time of the machine element 1b, loads applied, rotational speeds and temperatures during operation, and the frequency of uses.

The display 34 is preferably e.g. an ordinary liquid crystal display. If the check items 51 include audio data, besides text data and drawing data, the display 34 may be provided with a speaker. Users perform examination according to the check items 51 indicated on the display 34.

The input unit 35 has to be one which allows the entry of at least YES and NO, and may be a touch panel, buttons, or a keyboard. Preferably, the input unit is one through which numerals and letters can be entered. Through the input unit 35, users enter information on whether or not the inspection carried out according to the check items 51 is correct or the result information 53, which is e.g. numeral values representing measurement results.

During the diagnosis on whether or not the machine element is reusable, when the result information 53 is entered through the input unit 35, the control unit 31 performs as a determination unit 36 which determines whether or not requirements are satisfied according the standard of determination 52a which has been read beforehand or which is read at that moment. If there is only one check item 51, the determination unit determines that the machine element 1b is reusable if the requirements are met and indicates this result of determination 54a on the display 34.

If there are a plurality of check items 51, after entering the result information 53 for one of the check items 51, the control unit indicates the next check item 51 on the display, and waits until the result information 53 for the next check item is entered. This operation is carried out for all of the check items 51. The operation as the determining unit 36 may be carried out simultaneously for all of the check items 51 after the result information 53 for all of the check items 51 has been entered, or may be carried out every time the result information 53 for one check item 51 is entered.

If the display 34 has an enough display area, a plurality of the check items 51 may be indicated simultaneously to wait for the entry for the respective check items. But in order to prevent entry errors, the check items 51 are preferably indicated one at a time.

The control unit 31 determines, as the determination unit 36, about the use information 55 based on the standard of history determination 56.

When the result information 53 regarding all of the check items 51, as well as the use information 55, is entered, and the control unit 31 finishes determination regarding the standard of determination 52a and the standard of history determination 56, the control unit 31 determines, based on all of the values obtained, whether or not the machine element 1b diagnosed is reusable. Basically, the control unit 31 determines that the machine element is non-reusable if even one of the requirements is met. But according to the type of the machine element and what is diagnosed, the control unit may determine that the machine element is reusable only if the machine element satisfies the requirements of more than a predetermined number of check items. This varies with the function, characteristics and properties of the machine element 1b. For example, the control unit may be programmed such that it determines that a certain machine element is passable if it satisfies a predetermined number of the items listed in the model number-item correspondence table.

The thus obtained determination result 54a, i.e. the determination on whether or not the machine element is reusable, is indicated on the display 34, and the diagnosis ends. The user thus knows whether or not the machine element 1b is reusable.

Thereafter, if necessary, the result information 53, determination result 54a and use information 55 may be written into the IC tag 11 of the machine element 1b. By writing the determination result 54a, anyone can check at any time whether or not the machine element 1b has been determined to be reusable. This especially helps prevent erroneous use of a machine element 1b that has been determined to be non-reusable. By recording the result information 53 and the use information 55, this information remains as the use history and diagnosis history. Thus, the later diagnosis on whether or not the machine element is reusable can be carried out based on the values contained in the above information.

When calculating the remaining life, when the result information 53 (including the use information 55) is entered through the input unit 35, this information is assigned to a determination equation 52b which has been read in beforehand or is read in at that point, to calculate the remaining life.

If the display 34 has an enough display area, a plurality of the check items 51 may be indicated simultaneously to wait for the entry for the respective check items. But in order to prevent entry errors, the check items 51 are preferably indicated one at a time.

The thus calculated remaining life 54b is indicated on the display 34, and the diagnosis ends. The user thus knows the remaining life 54b of the machine element 1b. The remaining life may be defined as the time period during which the machine element is actually usable, or as the remaining life when the machine element is not used at all, depending on the kind of the machine element 1b.

Thereafter, if necessary, the result information 53, remaining life 54b and use information 55 may be written into the IC tag 11 of the machine element 1b. Especially by writing the remaining life 54b, anyone can check at any time how longer the machine element 1b can be used. This especially helps prevent erroneous use of a machine element 1b that has been determined to have zero remaining life 54b is thus non-reusable. By recording the result information 53 and the use information 55, this information remains as the use history and diagnosis history. Thus, the later diagnosis on whether or not the machine element is reusable can be carried out based on the values contained in the above information.

Further functions are described with reference to both the determination on whether or not the machine element is reusable and the calculation of the remaining life. If the inspection device 30 has a network functional unit 37, the memory 33 may be a volatile one. In this case, instead of normally storing the check items 51, standard of determination 52a, determining equations 52b, standard of history determination 56, etc. in the memory, the identification information 50 for the selected machine element is preferably transmitted from the network functional unit 37 to an external check item control database 71 through the network 81, thereby downloading the check items 51, standard of determination 52a, standard of history determination 56, etc. which all correspond to the transmitted identification information 50 and temporarily storing these data in the memory 33, every time determination similar to the above is to be performed. With this arrangement, diagnosis is possible for machine elements 1b manufactured after the inspection device 30 has been sold to a user. The network functional unit 37 may be an internet connecting function through a wired or wireless LAN, or a mobile communication function, provided it can access the check item control database 71, which exists in the network. The check item control database 71 is preferably be run by a manufacturer or a distributor of the machine element 1b so that the manufacturer or distributor services so that the data in the database 71 is provided to the purchaser through the manufacturer or the distributor.

If the inspection device 30 has the network functional unit 37, use information 55, determination result 54a, remaining life 54b, etc., as well as the identification information 50, are preferably transmitted to a machine element control database 61, which exists in the network 81, while determination is being made on whether or not a machine element is reusable, or after this determination. Upon receipt of these data, the machine element control database 61 stores these data in a table so that they can be read out whenever necessary. Thus, by searching the machine element control database 61, e.g. the firm where the user is working can obtain information on whether any particular machine element 1b they own is currently being used and if it is, how frequently it is used.

The above-mentioned databases are computer software stored and run in a server usable on the network 81.

As another function, the inspection device 30 preferably contains a battery 39 so that the inspection device can determine whether or not machine elements are reusable at a location near the site where the machine elements are actually used and where no electric power is available.

During determination on whether or not a machine element 1b is reusable, if the machine element 1b includes a plurality of component parts, it is possible to refer to the check items 51 for all of these components based on the identification information 50 stored in the IC tag 11, so that it is possible to determine whether or not the machine element is reusable as a whole based on determinations of the individual component parts.

In calculating the remaining life of a machine element 1b, if the machine element 1b includes a plurality of component parts, it is possible to refer to the check items 51 for all of these components based on the identification information stored in the IC tag 11, so that it is possible to calculate the remaining life 54b of the machine element as a whole based on determinations of the individual component parts, or it is also possible to calculate remaining lives 54b of the individual component parts. With the latter arrangement, it is possible to replace only a component part or parts whose remaining lives have decreased to zero with a new one or ones.

In calculating the remaining life, according to the characteristics of the machine element 1b, the remaining life 54b may be calculated in multiple stages instead of using a single determining equation 52b. In particular, after calculating a first determining equation 52b for diagnosing the state of the machine element 1b, check items 51b for a second determining equation 52b selected according to the result of the above calculation, and this second equation 52b is newly read in, and the check items 51b are indicated on the display 34. Then, after receiving the result information 53 and the use information 55, the determining equation 52b is calculated. In other words, the above determining equations include not only the determining equation 52b for directly calculating the remaining life 54b but also a determining equation used in a determining step before calculating the remaining life 54b and branching of conditions for selecting the equation to be used. It is thus possible to use different equations while the remaining life is sufficiently long and not very long so that the remaining life can be calculated with higher accuracy when the remaining life is short. It is also possible to change the contents of inspection according to the nature of the problems the machine element 1b is encountering.

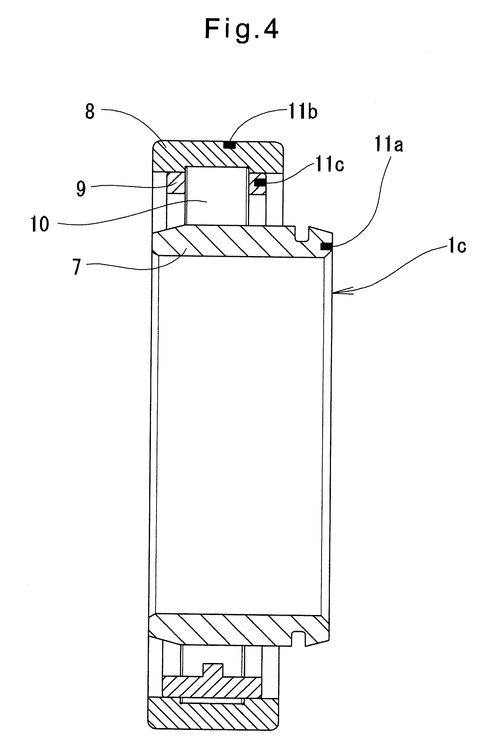

Now an embodiment is described of which the machine element 1b includes a plurality of component parts, and IC tags are used for the individual component parts. Fig. 4 shows a sectional view of a machine element 1c, which is a roller bearing including an inner race 7, an outer race 8, a retainer 9, and rolling elements 10. IC tags 11a, 11b and 11c are attached to the inner race 7, outer race 8 and retainer 9, respectively.

In determining whether or not the machine element 1c is reusable, the inspection device 30 may first read all the identification information 50a to 50c stored in the respective IC tags 11a to 11c. Since the IC tag 11c is located between the inner race 7 and the outer race 8, it is difficult to read out the information from the IC tag 11c. Thus, in order to read out information from the IC tag 11c, it is necessary to disassemble the roller bearing. The inspection device 30 may keep, in its memory 33, a correspondence table showing the relationship between the identification information 50a to 50c, stored in the respective IC tags 11a to 11c, and the check items. Or otherwise, this table may be downloaded from the network and temporarily stored in the memory. With this table stored in the memory, the inspection device 30 indicates the check items 51 for the respective identification information groups 50a to 50C one after another, and receives all of the result information 53 and temporarily stores it in the memory 33. When all of the result information 53 has been temporarily stored in the memory 33, determination may be made on the thus stored result information, or the remaining life 54b is calculated by assigning the result information 53 to the determining equation 52b. Needless to say, in either case, the determination may be additionally made on the use information 56 based on the standard of history determination 56. The result of determination 54a, which is obtained based on all of these determinations, as well as the calculated remaining life 54b, is indicated on the display 34. If necessary, use information 55 and the result of determination 54a are written into the respective IC tags 11a to 11c. Then, if the bearing is determined to be reusable, it is reassembled and reused.

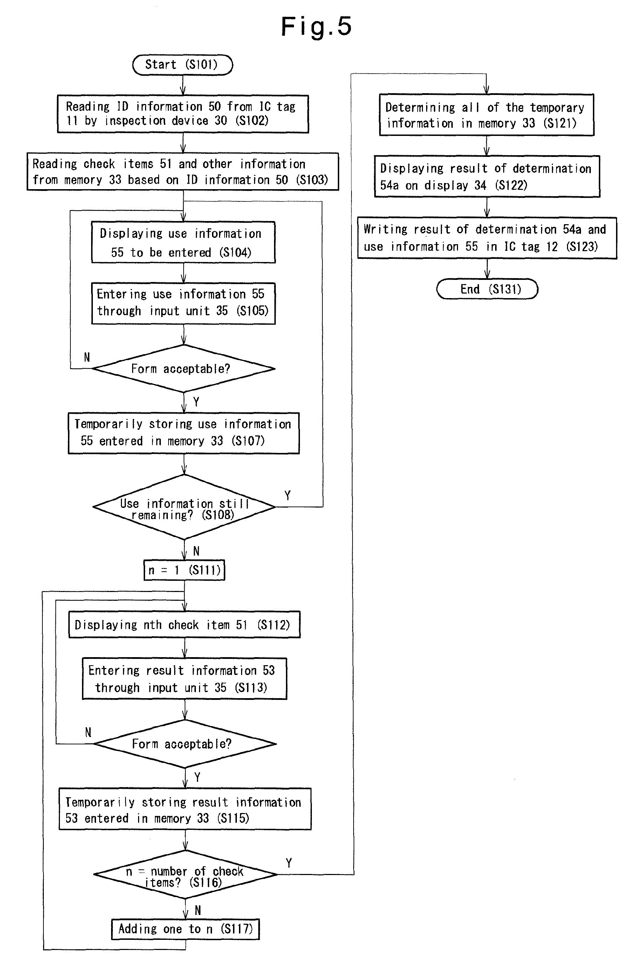

Now referring to the flowchart of Fig. 5, description is made of the diagnostic method according to the present invention for determining whether or not a machine element is reusable.

First (Step 101), a user moves the inspection device 30 close to the IC tag 11 of the machine element 1b so that the inspection device can read the identification information 50 (Step 102). The control unit 31 searches the table in the memory 33 using the identification information 50 read from the IC tag as a search key, and reads out the check items 51, standard of determination 52a, and standard of history determination 56 (Step 103). The check items 51 include information on use information 55, which needs to be entered.

The control unit 31 displays a message prompting entry of the use information 55 on the display 34 (Step 104). When use information 55 corresponding to the information on the display is entered (Step 105), the control unit 31 determines whether or not this information is free of formal defects (Step 106). For example, if numerical data are entered through the input unit 35 in response to an enquiry requesting a YES or NO answer, or if decimal points or negative numbers are included in data which is supposed to include only natural numbers, the control unit 30 determines that the entry is in error. In this case, the program returns to Step 104. If the entered data are free of formal problems, the control unit 31 temporarily stores the use information 55 in the memory 33 (Step 107). If the use information 55 contains a plurality of information groups (Step 108), the above steps (Steps 104 to 107) are repeated for the next use information group.

Next (Step 111), the control unit 31 indicates the contents of the first one of the check items 51 on the display 34 (Step 112). According to the contents on the display 34, the user performs visual inspection and/or inspection using measuring devices. Upon completion of the inspection, the user enters the result information 53 through the input unit 35 (Step 113). The control unit 31 determines whether or not the entered information is free of formal problems in the same manner as above (Step 114). If it is, the control unit 31 temporarily stores the result information 53 in the memory 33 (Step 115). When the n value reaches the number of the check items 51, the inspection ends (Step 116). If not (Step 117), the next check item 51 is displayed (Steps 112 +).

When the inspection ends (S116), the control unit 31 (determination unit 36) reads out the use information 55 and result information 53 temporarily stored in the memory 33, and checks whether or not all of the items satisfy the requirements, referring to the standard of history determination 56 and the standard of determination 52a (Step 121). If all the results meet the requirements, the result of determination 54a is "positive". Otherwise, the result of determination 54a is "negative". The control unit 31 displays the thus obtained result of determination 54a on the display (Step 122). Upon completion of the determination, the user moves the inspection device 30 close to the IC tag 11 to write the result of determination 54a and the use information 55 into the IC tag 11 (Step 123).

This completes the determination on whether or not one machine element 1b is reusable (Step 131).

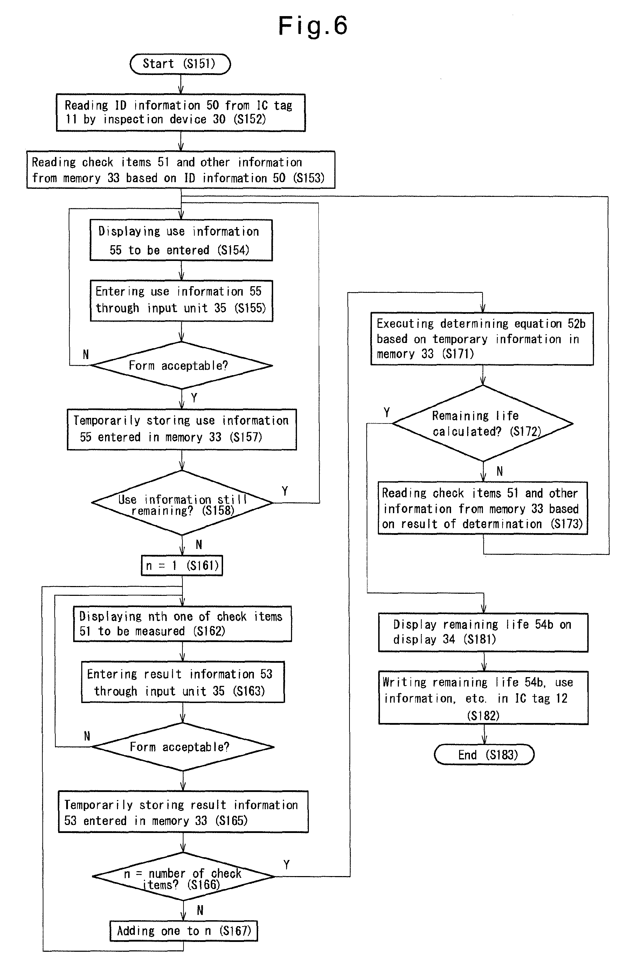

Next, referring to the flowchart of Fig. 6, description is made of the diagnostic method according to the present invention for calculating the remaining life.

First (Step 151), the user moves the inspection device 30 close to the IC tag 11 of the machine element 1b so that the inspection device can read the identification information 50 from the IC tag (Step 152). The control unit 31 searches the table in the memory 33 using the identification information 50 read from the IC tag as a search key, and reads out the check items 51 and the determining equation 52b (Step 153). The check items 51 include information on use information 55, which needs to be entered.

The control unit 31 first displays a message prompting, among the check items 51, entry of the use information 55 on the display 34 (Step 154). When use information 55 corresponding to the information on the display is entered (Step 155), the control unit 31 determines whether or not this information is free of formal defects (Step 156). For example, if decimal points or negative numbers are included in data which is supposed to include only natural numbers, the control unit 30 determines that the entry is in error. In this case, the program returns to Step 154. If the entered data are free of formal problems, the control unit 31 temporarily stores the use information 55 in the memory 33 (Step 157). If the use information 55 contains a plurality of information groups (Step 158), the above steps (Steps 154 to 157) are repeated for the next use information group.

Next (Step 161), the control unit 31 indicates the contents of the first one of the check items 51 other than the use information 55, i.e. the check items 51 which require actual measurement, on the display 34 (Step 162). According to the contents on the display 34, the user performs inspection using a balance, a scale and various other measuring devices and instruments. Upon completion of the inspection, the user enters the result information 53 through the input unit 35 (Step 163). The control unit 31 determines whether or not the entered information is free of formal problems in the same manner as above (Step 164). If it is, the control unit 31 temporarily stores the result information 53 in the memory 33 (Step 165). When the n value reaches the number of the check items 51, the inspection ends (Step 166). If not (Step 167), the next check item 51 is displayed (Steps 162 +).

When the inspection ends (S166), the control unit 31 reads out the use information 55 and result information 53 temporarily stored in the memory 33, and assign this information to the determining equation 52b to obtain a result (Step 171). If the result is the ultimate remaining life 54b (Step 172), the control unit 31 displays this value on the display (Step 181). After the remaining life is displayed, the user may move the inspection device 30 close to the IC tag 11 to write the remaining life 54b, use information 55, result information 53, etc. into the IC tag (Step 182). This completes the inspection (Step 183).

If the result obtained from the determining equation 52b is not the final remaining life 54b but indicates a stage of determining the status of the machine element 1b (Step 172), the control unit 31 reads out, from the memory 33, check items 51 corresponding to the result of determination of the status of the machine element 1b, as well as the next determining equation 52b (Step 173), and performs similar steps for the respective check items 51 (Steps 154+ or Steps 162+, depending on the check items).

Next, an example is shown in which the machine elements 1b are rolling bearings, and in which the remaining lives are calculated in different ways according to the specific situation. In this example, the rolling bearings as the machine elements are those used in pumps and fans used as accessories in chemical plants, ironworks and electric power plants, and those used in motors for driving these accessories. The loads applied to these rolling bearings are very low, i.e. 5% or less of the rated load, so that these bearings are free of metal fatigue as long as they are being used in a normal manner. Thus, their remaining lives will never significantly decrease, unless they suffer from "flaking due to stress concentration" at protruding portions of impressions formed due to inclusion of solid foreign matter, or suffer from "increased vibration" due to partial disappearance of grease film due to inclusion of water, and the resulting increase in surface roughness of the raceways of the rolling bearings. The remaining lives are calculated in different ways for the respective cases. Thus, in this example, check items 51 and a determining equation 52b are necessary which are used in a determining stage in which the status of the rolling bearing as the machine element 1b to be diagnosed is determined. Its remaining life is calculated if a specific requirement is met in this determining stage.

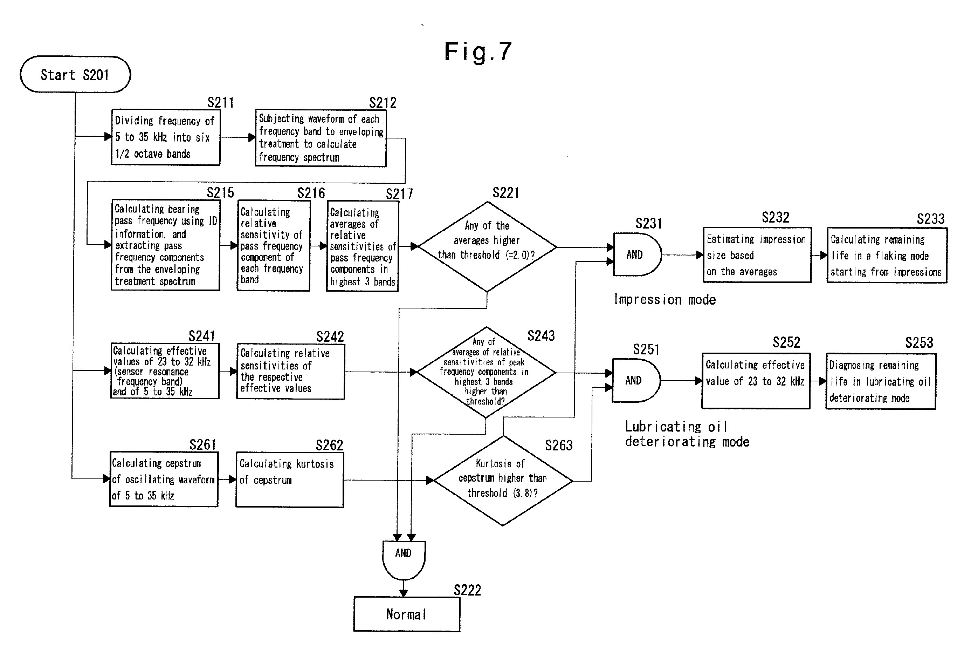

The check items 51 used in this determining stage include calculating a signal oscillation using an acceleration sensor, and indicating the signal oscillation on the display 34 to obtain its result information 53. Fig. 7 shows a typical determining equation 52b as a logic flow for making determination based on the result information 53.

First, the oscillation waveform of 5 kHz to 35 kHz are divided into six 1/2 octave bands (Step 211), and the waveform of each frequency band is subjected to envelope treatment to calculate the frequency spectrum (Step 212).

Next, the bearing pass frequency is calculated based on the identification information 50 of the IC tag 11 corresponding to the rolling bearing to be diagnosed. Then, three pass frequency components ($f_{inner}$, $f_{outer}$ and $f_{ball}$) are extracted from the envelop treatment spectrum of each frequency band (Step 215). The bearing pass frequencies are calculated differently for the respective model numbers of rolling bearings, and are each calculated by assigning a variable peculiar to the identification information 50. Next, the relative sensitivities (ratios to the normal) of the extracted pass frequency components of each frequency band are calculated (Step 216) to obtain the averages of the relative sensitivities of the pass frequency components of the highest three bands (Step 217). These calculations are performed independently of the three individual components $f_{inner}$, $f_{outer}$ and $f_{ball}$ to determine whether or not any of the averages of the three components ($f_{inner}$, $f_{outer}$ and $f_{ball}$) of the highest three bands is higher than a threshold (=2.0) (Step 221). If not, the control unit assumes that the rolling bearing is normal (Step 222). These steps represent the initial stage of the determining equation 52b.

If the threshold is exceeded by any of the above averages, the control unit determines that the rolling bearing is in a flaking mode starting from impressions (Step 231), estimates the size of the impressions based on the relative sensitivity averages of the highest three bands (Step 232), determines the remaining life in the flaking mode starting from impressions, and calculates the remaining life based on the below equation (1) (Step 233).

In equation (1), P is the radial load applied to the bearing during the test, C is the basic dynamic load rating of the bearing, and n is the revolutions per minute (rpm). $L_{10h}$ is the basis load rating life under JIS B1518 1992, i.e. the period of time during which 90% of a group of identical bearings can rotate without suffering from flaking due to rolling fatigue when they are rotated under the same conditions.

[Equation 1]

$$L_{10h} = \frac{10^6}{60n}\left(\frac{C}{P}\right)^3 \quad (1)$$

The 10% life $L_{10}$ when the life test data is assigned to the Weibull distribution is given by the below equation (2) with reference to $L_{10h}$. Since the constants a and b can be developed by collinear approximation based on past experience as shown in equation (3) with reference to the diameter d ($\mu$m) of impressions. Equation (4) can thus be developed into equation (4).

[Equations 2]

$$log\ (L_{10}/L_{10h})\ =a\ log\,(P/C)+b \qquad (2)$$

$$log\ (L_{10}/L_{10h})\ =0.0038d\ log\,(P/C)-0.272log\,(d)+0.416 \qquad (3)$$

$$L_{10}\ =\ L_{10h}\ \times\ 10^{(0.0038d\ log\,(P/C)-0.272log\,(d)+0.416)} \qquad (4)$$

That is, according to the present invention, in the check items 51, the control unit indicates a request to enter the above values P, C, n and d as the use information 55 on the display 34. The diameter of the impressions is obtainable by treating the oscillation waveform. When these values are entered through the input unit 35, the control unit 31 assigns these values to the above equations (1) and (2), as the determining equations to calculate the $L_{10}$ life, i.e. the remaining life 54b, and indicates the remaining life on the display 34.

Next, description is made of the flow of calculation of the remaining life if the lubricating oil of the rolling bearing deteriorates. In particular, the control unit calculates the effective value in the sensor resonance frequency band of 23 to 32 kHz (Step 241), calculates the relative sensitivity of the effective value in the 23 to 32 kHz band (Step 242), and determines whether or not the relative sensitivity of the effective value in the 23 to 32 kHz band is higher than a threshold (=1.5) (Step 243). If not, the control unit assumes that the bearing is normal (Step 222). According to the present invention, once the values measured by the acceleration sensor are entered as the result information 53, the control unit 31 automatically performs calculations and determinations in Steps 241 to 243 and up to Step 222 as determining equations 52b.

If the relative sensitivity is higher than the threshold (Step 251), the control unit determines that the bearing is in a mode in which lubricating oil has deteriorated. Thus, the control unit estimates the degree of deterioration of the lubricating oil based on the effective value in the 23 to 32 kHz band (Step 252), and based on the result thus obtained, calculates the remaining life (Step 253). In particular, the control unit first calculates the calculated life of Booser equation ($L_{hb}$) based on equation (5) below.

[Equation 3]

$$\log\left(L_{hb}\right)=-2.30+\frac{2450}{273+t}-0.301\left(S_{G}+S_{N}+S_{W}\right) \quad (5)$$

In equation (5), t is the temperature (°C) of the bearing outer race, $S_{G}$ is the half-life subtraction coefficient according to the grease composition, and $S_{N}$ and Sw are the half-life subtraction coefficient according to the number of revolutions and the half-life subtraction coefficient according to the load and speed, respectively. SG is a value peculiar to the grease used and is stored in the table of the memory 33 so as to correspond to the identification information 50. $S_{N}$ and Sw are calculated based on equations (6) and (7) below, respectively.

[Equation 4]

$$S_{N}=0.864\times dn/(dn_{L}) \qquad (6)$$

$$S_{W}=0.61ndP/Cr^{2} \qquad (7)$$

In these equations, d (mm) is the inner diameter of the bearing, nL is the maximum permissible rotational speed (in rpm) in the catalogue, P (lbf) is the load applied, and Cr (lbf) is the basic dynamic load rating. These values are assigned to the equations (5) to (7), which form determining equations 52b, as variables derived from the use information 55 and the identification information 50. Then the remaining life is calculated based on the below equation (8).

[Equation 5]

$$L = L_{hb} \times V_r^{-4.44} \qquad (8)$$

A separate determination route other than the above-mentioned step may be prepared and indicated on the display 34 so that this route can be selected through the input unit 35. The control unit calculates the cepstrum of the 5 to 35 kHz oscillation waveform from the spectrum measured by the acceleration sensor (Step 261), calculates the kurtosis of the cepstrum (Step 262), and determines whether or not the kurtosis is higher than a threshold (=3.8) (Step 263). If the kurtosis is higher than the threshold, the control unit calculates the remaining life in the peel mode starting from impressions in the above manner (Steps 231 to 233), and otherwise calculates the remaining life in the mode in which lubricating oil has deteriorated (Steps 251 to 253).

Next, specific machine elements carrying IC tags are described which can be determined whether or not they are reusable and of which the remaining life can be determined by the diagnostic method of the present invention.

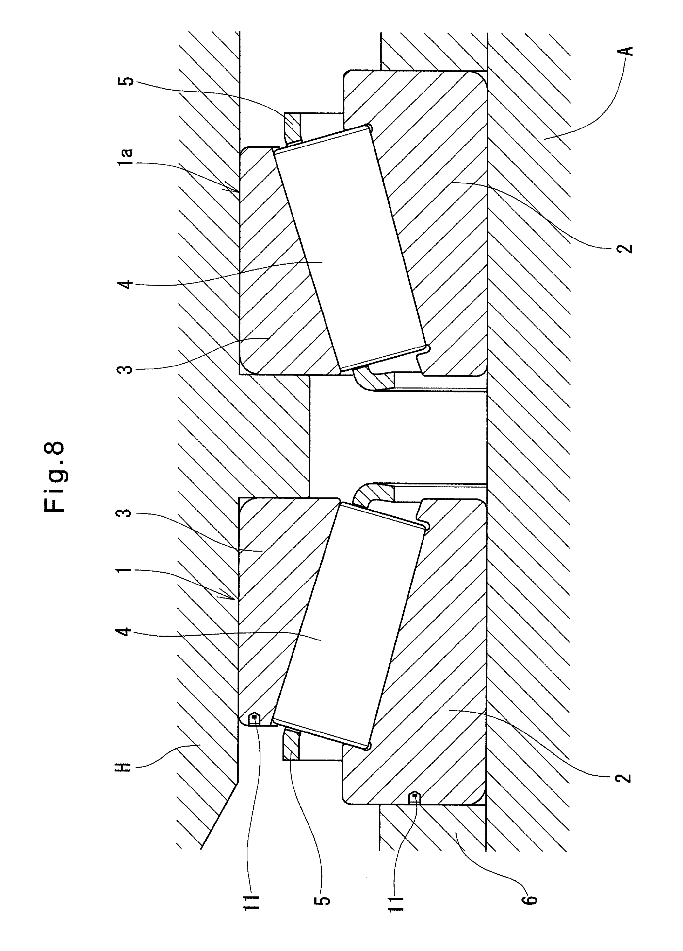

First, a rolling bearing 1 carrying an IC tag or tags according to a first embodiment is described. Fig. 8 shows how the rolling bearing 1 with IC tags according to the first embodiment are used. The rolling bearing 1 shown is a tapered roller bearing including an inner race 2, an outer race 3, and a plurality of tapered rollers 4 and an annular retainer that are disposed between the inner race 2 and the outer race 3. Two IC tags 11 of the non-contact communication type are mounted to respective end surfaces of the inner race 2 and the outer race 3 on the same side. This tapered roller bearing 1 and another similar tapered roller bearing 1a, carrying no IC tags, are mounted between a housing H and a rotary shaft A.

A presser member 6, located leftwardly (in Fig. 8) of the inner race 2 of rolling bearing 1, presses the inner race 2 rightwardly, thereby applying a preload to the bearing 1. Thus, in order to read out information stored in the IC tag 11 mounted to the inner race 2, it is necessary to e.g. remove the presser member 6 to expose the IC tag 11, and then move the inspection device 30, which is a reader/writer, close to the IC tag 11. In order to read information stored in the IC tag 11 mounted to the outer race 3, the rolling bearing 1 and the surrounding members are disassembled until the inspection device 30 can be moved sufficiently close to the IC tag 11, and the inspection device 30 is moved close to the IC tag 11 to read the information therein. Needless to say, if the inspection device 30 can be moved sufficiently close to the IC tag 11 on the outer race, it is not necessary to disassemble the roller bearing or any other parts.

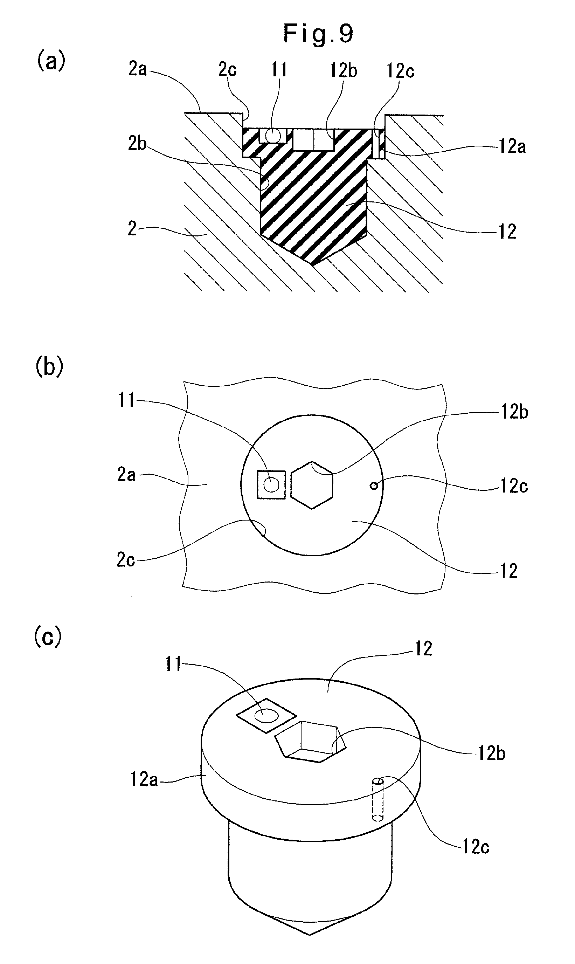

Fig. 9 shows a mounting arrangement for mounting the IC tag 11 to the inner race 2. This mounting arrangement includes a casing 12 inserted in a circular mounting hole 2b formed in a mounting surface (an end surface) 2a of the inner race 2 and fixed in position by means of an adhesive, with the IC tag 11 mounted in the casing 12 (see Fig. 9(a)). The adhesive is applied to the inner wall of the mounting hole 2b before inserting the casing 12 into the hole 2b. The casing 12 is made of a synthetic resin, which is less likely to interfere with the communication between the IC tag 11 and the reader/writer. The casing 12 is entirely inserted in the mounting hole 2b, which effectively prevents damage to the IC tag 11 and the casing 12 by being hit by foreign matter.

The mounting hole 2b of the inner race 2 has a countersunk portion 2c extending along the open edge of the hole 2b, and has a conical bottom (see Fig. 9(a)). The casing 12 has an outer contour complementary in shape to the inner wall of the mounting hole 2b. In particular, the casing 12 includes a lid portion 12a fitted in the countersunk portion 2c of the mounting hole 2b, and has a conical surface complementary in shape to the conical bottom of the mounting hole 2b, at the end of the casing 12 which is to be inserted first into the hole 2b (see Figs. 9(a) and 9(c)). With this arrangement, the casing 12 can be stably held in position in the mounting hole 2b.

The casing 12 has a hexagonal hole 12b formed in the center of the top surface of the lid 12a in which a tool such as a hexagonal wrench is engageable. The IC tag 11 is fitted in a recess formed in the top surface of the lid so as not to interfere with the hexagonal hole 12b. An air vent passage 12c extends axially through the lid 12a from its top to bottom surface near the radially outer periphery of the lid 12a through which air in the mounting hole 2b can be expelled when the casing 12 is inserted into the mounting hole 2b (Figs. 9(a) to 9(c)).

Since air in the mounting hole 2b can be expelled through the air vent passage 12c formed in the casing 12 when the casing 12, carrying the IC tag 11, is inserted into the mounting hole 2b and fixed in position by an adhesive. Thus, air scarcely remains in the mounting hole 2b. This in turn allows a larger amount of adhesive to remain in the mounting hole 2b, which prevents the casing 12 from coming out of the mounting hole 2b, and prevents rust on the inner race 2, which could deteriorate the outer appearance of the inner race.

In order to more reliably prevent separation of the casing from the mounting hole, the casing may be formed with an external thread on its outer periphery so that the casing is brought into threaded engagement with the mounting hole by driving the casing into the mounting hole.

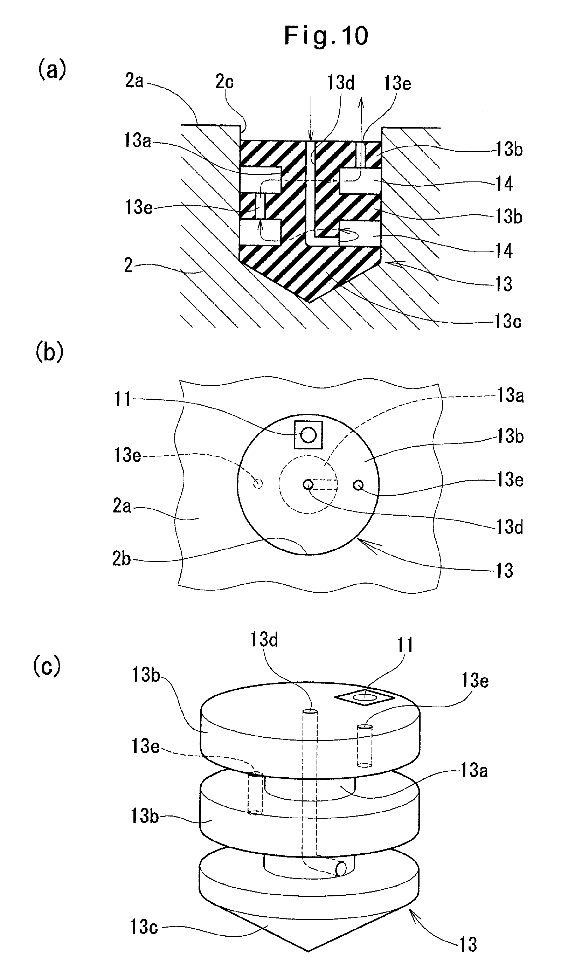

Figs. 10(a) to 10(c) shows the second embodiment, which is based on the first embodiment but differs from the first embodiment in that the mounting hole 2b of the inner race 2 has no countersunk portion and the casing 13 has such an outer contour that the casing can be snugly fitted in this mounting hole 2b. The casing 13 includes a shaft portion 13a located along the center axis of the mounting hole 2b, two flange portions 13b radially outwardly extending from the shaft portion 13a, and a leading end portion 13c having a conical surface similar to the one of the first embodiment at its end which is to be inserted first into the mounting hole. The IC tag 11 is received in a recess formed in the top end surface of one of the flanges 13b located closer to the opening of the mounting hole 2b.

The flanges 13b define two spaces in the mounting hole 2b that are spaced apart from each other in the depth direction of the mounting hole 2b and serve as adhesive reservoirs 14. The shaft portion 13a is formed with an adhesive introducing passage 13d through which adhesive can flow and which has an outlet port opening to the outer periphery of the shaft portion 13a so as to communicate with one of the adhesive reservoirs 14 that is located closer to the bottom of the mounting hole 2b. Air vent passages 13e axially extend through the respective flanges 13b. One of the air vent passages 13e located closer to the bottom of the mounting hole 2b is located diametrically opposite to the outlet port of the adhesive introducing passage 13d formed in the shaft portion 13a. The other air vent passage 13e, which is located closer to the opening of the mounting hole 2b, is located on the same side as the outlet port of the adhesive introducing passage 13d. Thus, as viewed from one side of the casing, the respective air vent passages 13e and the outlet port of the adhesive introducing passage 13d are arranged in a staggered manner in the axial direction on both sides of the center axis of the shaft portion 13a.

In the second embodiment, when adhesive is poured into the adhesive introducing passage 13d after the casing 13 has been inserted into the mounting hole 2b, the adhesive gradually fills the adhesive reservoirs 14 and the air vent passages 13e from the bottom side of the mounting hole 2b as shown by the arrows in Fig. 10(a). At the same time, the adhesive pushes out air in the mounting hole 2b. Thus, with this arrangement, by pouring adhesive into the adhesive introducing passage, it is possible to smoothly fill the mounting hole 2b with adhesive while at the same time smoothly expelling air.

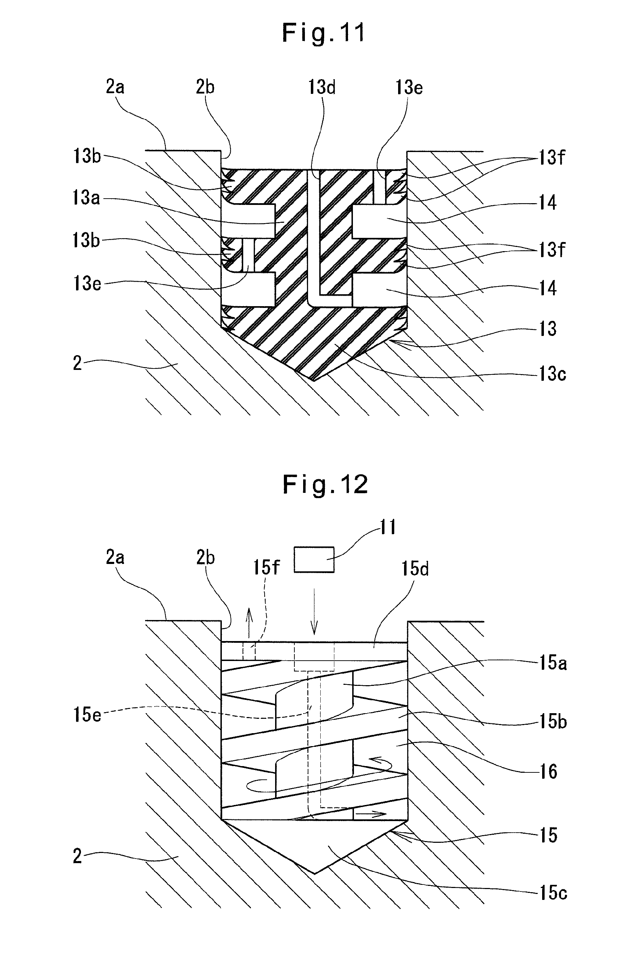

Fig. 11 shows a casing 13 which has an outer periphery slightly different in shape from that of the casing 13 of the second embodiment. This casing 13 has a plurality of annular protrusions 13f on the radially outer surface of each of the flanges 13b and the leading end portion 13c. The annular protrusions have their radially outer edges bent toward the opening of the mounting hole 2b to more reliably prevent separation of the casing 13 from the mounting hole 2b. As another way to prevent separation of the casing, the radially outer portion of the casing may be made of a shape memory alloy and formed into a shape such that the casing cannot be easily pulled out of the mounting hole at normal temperature and the casing can be deformed at low temperature such that the casing can be easily inserted into the mounting hole.

Fig. 12 shows the third embodiment, in which a casing 15 including a helical rib 15b provided on the outer periphery of the shaft portion 15a is inserted in an mounting hole 2b which is identical in shape to that of the second embodiment such that a helical adhesive reservoir 16 is defined between the outer periphery of the shaft portion 15a of the casing 15 and the inner wall of the mounting hole 2b.

The casing 15 is mounted in the mounting hole 2b with its shaft portion 15a located along the center axis of the mounting hole 2b and its helical rib 15b in contact with the inner wall of the mounting hole 2b. The casing further includes a leading end portion 15c similar to that of the second embodiment and located closer to the bottom of the mounting hole 2b than is the helical rib 15b, and a lid portion 15d located closer to the opening of the mounting hole 2b and closing the mounting hole 2b at its portion close to the opening of the mounting hole 2b. The shaft portion 15a is formed with an adhesive introducing passage 15e having an outlet port open to the outer periphery of the shaft portion 15a at its portion close to the bottom of the mounting hole 2b. The lid 15d is formed with an air vent hole 15f extending axially through the lid 15d.

In the third embodiment, when adhesive is poured into the adhesive introducing passage 15e of the casing 15 after the casing 15 has been inserted into the mounting hole 2b, the adhesive gradually fills the helical adhesive reservoir 16 and the air vent passage 15f from the bottom side of the mounting hole 2b as shown by the arrows in Fig. 12. At the same time, the adhesive pushes out air in the mounting hole 2b. Thus, with this arrangement too, by pouring adhesive into the adhesive introducing passage, it is possible to smoothly fill the mounting hole 2b with adhesive while at the same time smoothly expelling air, in the same manner as in the second embodiment. After filling adhesive, the IC tag 11 is fitted in a recess formed close to the inlet port of the adhesive introducing passage 15e of the lid 15d.

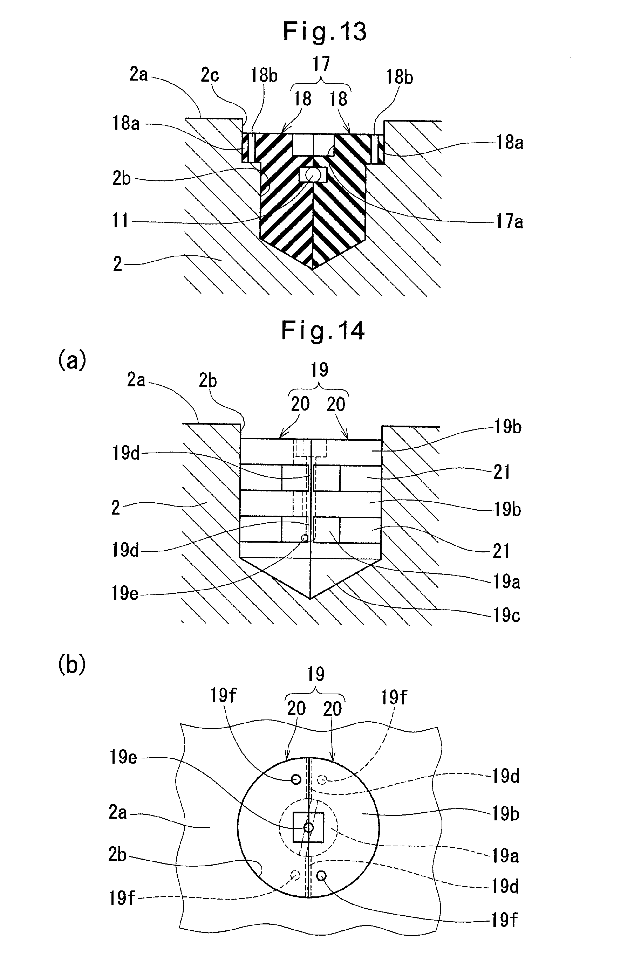

Fig. 13 shows the fourth embodiment, which is based on the first embodiment but differs from the first embodiment in that the casing 17 in which the IC tag 11 is to be received is made up of two separate case members 18. For reduced manufacturing cost, the two case members 18 are identical in shape and formed with a common mold.

The case members 18 each have a lid 18a formed with an air vent passage 18b axially extending therethrough near its peripheral edge. The case members 18 define two recesses along the abutment surfaces thereof when the case members 18 are brought into abutment with each other along the abutment surfaces. One of the two recesses that faces the opening of the mounting hole 2b is a hexagonal hole 17a. The IC tag 11 is received in the other of the two recesses, which is located toward the center of mounting hole.

The fourth embodiment is advantageous over the above-described other embodiments in that since the IC tag 11 can be sealed in the casing 17, the IC tag 11 never separates from the casing and thus from the mounting hole.

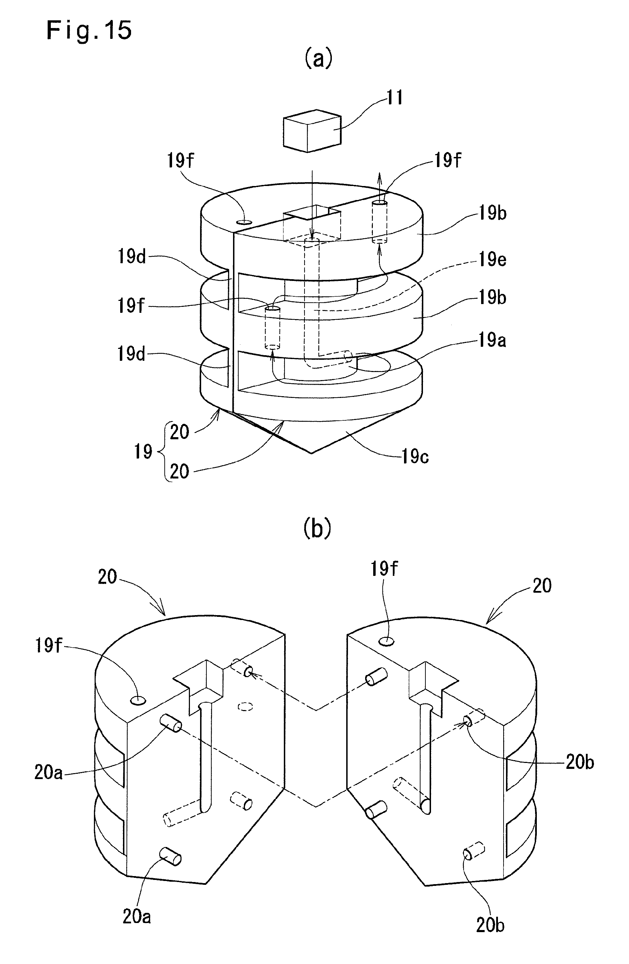

Figs. 14(a), 14(b), 15(a) and 15(b) shows the fifth embodiment, which is based on the second embodiment but differs from the second embodiment in that in order to reduce manufacturing cost, the casing 19 in which the IC tag 11 is to be received is made up of two separate case members 20. As in the fourth embodiment, the two case members 20 are identical in shape and formed by a common mold.

The casing 19 is similar to the casing 13 of the second embodiment in that it is basically made up of a shaft portion 19a, two flanges 19b, and a leading end portion 19c, but differs from the second embodiment in that partitioning walls 19d are provided between the flanges 19b and between the one of the flanges 19b located close to the bottom of the mounting hole 2b and the leading end portion 19c, dividing each of the adhesive reservoirs 21 into circumferentially spaced apart two portions. The shaft portion 19a has an adhesive introducing passage 19e having two outlet ports provided in the respective case members 20 at locations close to one of the partitioning walls 19d. Air vent passages 19f are formed in the respective flanges 19b. As viewed from one side of the casing, the air vent passages 19f and the outlet ports of the adhesive introducing passage 19e are arranged in a staggered manner in the axial direction on both sides of the center axis of the shaft portion 19a. With this arrangement, as shown by the arrows in Fig. 15(a), adhesive gradually fills the adhesive reservoirs 21 and the air vent passages 19f of the respective case members 20 from the bottom side of the mounting hole 2b. At the same time, the adhesive pushes out air in the mounting hole 2b. After filling adhesive, the IC tag 11 is fitted in a recess formed close to the inlet port of the adhesive introducing passage 19e.

As shown in Fig. 15(b), the case members 20 has abutment surfaces provided with a coupling arrangement including pins 20a and pin holes 20b formed on each abutment surfaces and arranged such that the pins 20a formed in the respective abutment surfaces can be simultaneously press-fitted into the respective pin holes 20 formed in the opposed abutment surfaces. Thus, the case members 20 can be coupled together by this coupling arrangement without using adhesive. A coupling arrangement other than the above-described coupling arrangement, such as a snap-fitting coupling arrangement, may be used to couple together the case members 20.

The mounting structures shown in Figs. 9 to 15 are all used to mount an IC tag to the inner race 2 of the rolling bearing 1. But any of these mounting structures can also be used to mount an IC tag 11 to the outer race 3 of the rolling bearing 1.

Any of the first to fifth embodiments can be used not only in a tapered roller bearing such as shown in Fig. 9 but also in other rolling bearings such as cylindrical roller bearings or ball bearings.

Now the sixth embodiment is described which is directed to a completely different mounting structure for mounting an IC tag to a rolling bearing 1 similar to the bearing shown in Fig. 9.

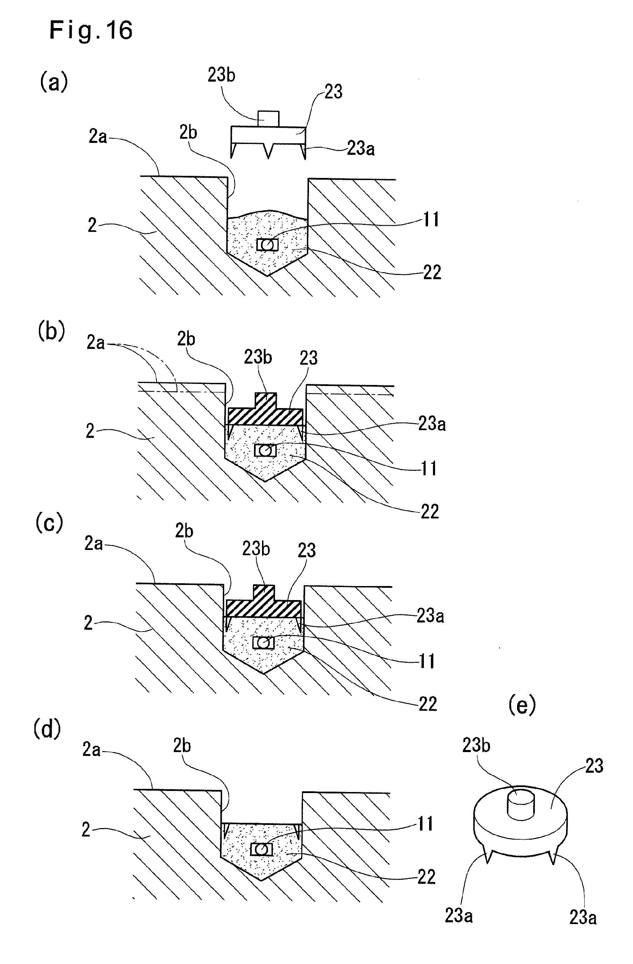

Fig. 16 shows how an IC tag 11 is mounted in position. In particular, first as shown in Fig. 16(a), an adhesive is poured into a mounting hole 2b with a conical bottom surface formed beforehand in a mounting surface (end surface) 2a of the inner race 2 of the bearing 1 (mounting object), with the IC tag 11 positioned in the mounting hole 2b, to form an adhesive layer 22 in which the IC tag 11 is trapped. The adhesive is poured such that it does not completely fill up the mounting hole 2b and the top surface of the adhesive layer 22 is located significantly spaced apart from the opening the mounting hole 2b toward the bottom of the mounting hole.

After pouring the adhesive and before the adhesive hardens completely, a lid 23 is inserted into the mounting hole 2b near its opening as shown in Fig. 16(b) to cover the top surface of the adhesive layer 22. The lid 23 is a disk-shaped member made of a synthetic resin because synthetic resin is less likely to interfere with communication between the IC tag 11 and the reader/writer. The lid 23 has a plurality of inwardly extending projections 23a on its inner bottom surface along its radially outer edge, and a tab 23b at the center of its outer top surface (see Fig. 16(e)). The lid 23 is pushed into the mounting hole until its projections 23b are stuck into the adhesive layer 22 and its inner flat surface is pressed against the top surface of the adhesive layer 22 so that the lid 23 cannot easily fall off the mounting hole 2b and also in order to flatten the top surface of the adhesive layer 22.

Next, in the state of Fig. 16(b), the mounting surface 2a of the inner race is ground to the position shown by phantom line in Fig. 16(b). This position is shown in Fig. 16(c). In the state of Fig. 16(b), in which the mounting surface 2a has not yet been ground, the lid 23 is sufficiently retracted from the mounting surface 2a such that the lid 23 is not ground when the mounting surface 2a is ground.

Finally, after the adhesive has hardened completely, the lid 23 is removed from the mounting hole 2b by holding its tab 23b. This state is shown in Fig. 16(d), in which the flat top surface of the adhesive layer 22, which has been formed by the inner surface of the lid 23, is exposed.

With this arrangement, since the lid 23 is inserted in the mounting hole 2b of the inner race near the opening of the mounting hole 2b to cover surface of the adhesive layer 22, in which the IC tag 11 is embedded, the lid 23 prevents grinding dust and other foreign matter from adhering to the surface of the adhesive layer 22. The lid 23 forms a flat top surface on the adhesive layer 22, which is exposed when the lid 23 is removed from the mounting hole 2b. Thus, the lid 23 improves the outer appearance of the adhesive layer 22.

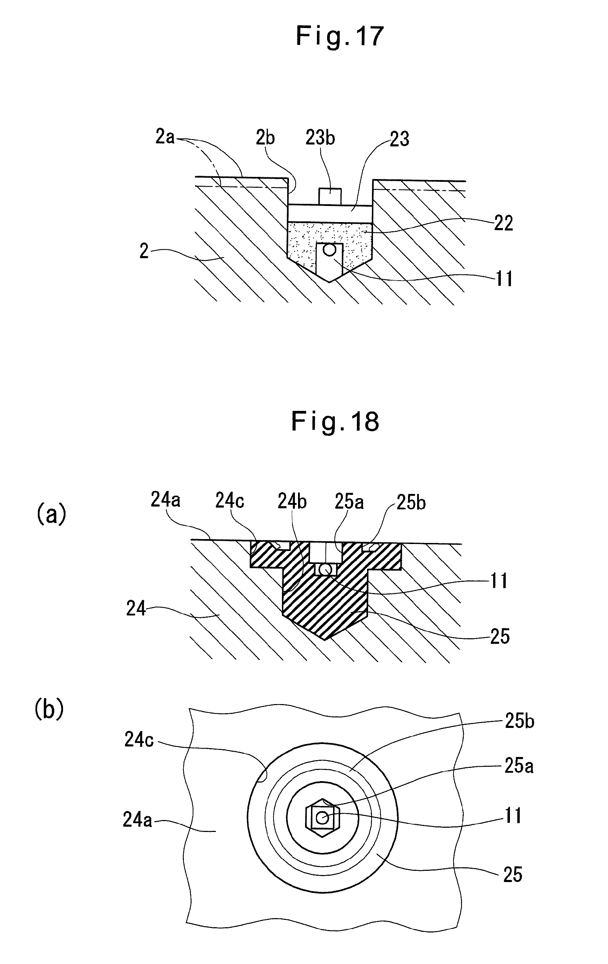

Fig. 17 shows the seventh embodiment, in which the IC tag 11 is mounted basically in the same manner as the sixth embodiment of Fig. 16(b). But this embodiment differs from the sixth embodiment in that the IC tag 11 has a conical bottom surface complementary in shape to the conical bottom surface of the mounting hole 2b. With this arrangement, when the IC tag 11 is mounted in position, the IC tag 11 can be more stably supported and fixed in position. The lid 23 has no inwardly extending projections and is press-fitted into the mounting hole 2b so as not to fall from the mounting hole 2b.

Like the lid of the sixth embodiment, the lid 23 of this embodiment also prevents foreign matter from adhering to the surface of the adhesive layer 22, and also forms a flat top surface on the adhesive layer, which is exposed when the lid 23 is removed, thus improving the outer appearance of the adhesive layer.

In the sixth and seventh embodiments, the lid 23 is removed after grinding the mounting surface 2a of the inner race 2. But the lid 23 may not necessarily have to be removed. If the lid 23 is not to be removed, no tab on the top surface of the lid is necessary, and it is also not necessary to press the lid against the surface of the adhesive layer.

The mounting structure of either of the embodiments of Figs. 16 and  17 is used to mount an IC tag to the inner race 2 of the rolling bearing 1. But either of these mounting structures can be used to mount an IC tag 11 to the outer race 3 of the rolling bearing 1 in the same manner as when an IC tag is mounted to the inner race 2.

Also, the mounting structure of either of the sixth and seventh embodiments can be used to mount an IC tag not only to a tapered roller bearing as shown in Fig. 9 but also to other rolling bearings such as cylindrical roller bearings and ball bearings.

Figs. 18(a) and 18(b) show an example in which a casing 25 in which an IC tag 11 is received is inserted into and fixed in position in a mounting hole 24b formed in a mounting surface 24a of a mounting object 24. This mounting structure has an arrangement for preventing foreign matter from adhering to the surface of the IC tag 11. Specifically, the mounting hole 24b has a countersunk portion 24c, and the casing 25 has a portion configured to be fitted in the countersunk portion 24c. A hexagonal hole 25a is formed in the center of the top surface of the casing 25. The IC tag 11 is received in a recess formed in the bottom of the hexagonal hole 25a. The casing 25 is further formed with an annular groove 25b to surround the hexagonal hole 25a. Any foreign matter such as grinding dust that approaches the casing 25 from around the mounting hole 24b falls into and is trapped in the annular groove 25b, and thus is prevented from adhering to the surface of the IC tag 11, located in the recess formed in the bottom of the hexagonal hole 25a.

DESCRIPTION OF THE NUMERALS

1. Rolling bearing
1a. Rolling bearing
1b. Machine element
1c. Machine element
2. Inner race
2a. Mounting surface
2b. Mounting hole
2c. Countersunk portion
3. Outer race
4. Tapered roller
5. Retainer
6. Presser member
7. Inner race
8. Outer race
9. Retainer
10. Rolling element
11. 11a. 11b. 11c. IC tag
12. 13. 15. 17. 19. Casing
12a. 15d. Lid
12b. 17a. Hexagonal hole
12c. 13e. 15f. 18b. 19f. Air vent passage
13a. 15a. 19a. Shaft portion
13b. 19b. Flange
13c. 15c. 19c. Leading end portion
13d. 15e. 19e. Adhesive introducing passage
13f. Protrusion
14. 16. 21. Adhesive reservoir

15b. Blade
18. 20. Case member
18a. Lid
19d. Partitioning wall
20a. Pin
20b. Pin hole
22. Adhesive layer
23. Lid
23a. Projection
23b. Tab
24. Mounting object
24a. Mounting surface
24b. Mounting hole
24c. Countersunk portion
25. Casing
25a. Hexagonal hole
25b. Annular groove
30. Inspection device
31. Control unit
32. Communication unit
33. Memory
34. Display
35. Input unit
36. Determination unit
37. Network functional unit
39. Battery
41. Communication unit
42. Memory
43. Control unit
50. 50a. 50b. 50c. Identification information
51. Check item
52a. Standard of determination
52b. Determining equation
53. Result information
54a. Result of determination
54b. Remaining life
55. Use information
56. Standard of history determination
61. Machine element control database
71. Check item control database
81. Network
H. Housing
A. Rotary shaft

**Claims**

1. A method of diagnosing a machine element, comprising:

mounting at least one IC tag to the machine element, wherein the IC tag stores identification information on at least one of the kind of the machine element, the time when the machine element was manufactured, a production lot of the machine element, and a production history of the machine element such that the identification information is electromagnetically readable from outside;
preparing an inspection device comprising a communication unit which can read the identification information stored in the IC tag, a memory which stores at least one check item corresponding to the identification information, and at least one of a standard of determination for the check item and a determining equations for calculating a remaining life based on result information for the check item, a display capable of displaying the check item and at least one of a result of determination and the remaining life, an input unit through which the result information for the check item can be entered, and a control unit for determining whether or not the machine element is reusable, thereby obtaining the result of determination, by comparing the result information entered through the input unit with the standard of determination, or calculating the remaining life using the determining equation; and

determining, using the inspection device, whether or not the machine element is reusable after the machine element has been used;

wherein in determining whether or not the machine element is reusable using the inspection device, the identification information of the machine element is read from the IC tag by the communication unit; inspection is carried out based on the check item after the check item has been displayed on the display; the result information, which is a result of the carrying out the inspection item, is entered through the input unit; and determination is made on whether or not the machine element is reusable, or the remaining life is obtained, on the spot, based on at least one of the result of determination and remaining life, which are displayed on the display.

2. The method of claim 1, wherein the at least one check item comprises a plurality of check items; wherein the inspection device is configured such that while a first one of the check items is displayed on the display, the result information corresponding to the first one of the check items can be entered, a second one of the check items is displayed on the display after the result information for the first one of the check items has been entered, and after the result information for all of the check items has been entered, determination is made on all of the standards of determination for all of the check items, thereby obtaining the result of determination.

3. The method of claim 1 or 2, wherein the inspection device is configured such that use information including at least either of a use time and use conditions of the machine element can be entered after the communication unit reads the identification information, wherein the memory stores a standard of history determination used as a standard in determining whether or not the machine element is reusable depending on the use information, and wherein in determining whether or not the machine element is reusable, the control unit also compares the use information with the standard of history determination, thereby obtaining the result of determination.

4. The method of claim 1, wherein the at least one check item comprises a plurality of check items including inputs of a use history of the machine element, and the result information includes use information for the use history.

5. The method of claim 1 or 4, wherein the at least one check item comprises a plurality of check items, wherein the display is capable of displaying the plurality of check items, wherein the inspection device is configured such that the result information corresponding to the plurality of check items can be entered through the input unit, and wherein the determining equation is based on the result information corresponding to the plurality of check items.

6. The method of claim 1 or 4, wherein the at least one check item comprises a plurality of check items; wherein the inspection device is configured such that while a first one of the check items is displayed on the display, the result information corresponding to the first one of the check items can be entered, a second one of the check items is displayed on the display after the result information for the first one of the check items has been entered, and after the result information for all of the check items has been entered, the determining equation is calculated based on the result information for all of the check items, thereby obtaining the remaining life.

7. The method of any of claims 1 to 6, wherein after determining whether or not the machine element is reusable or after calculating the remaining life, at least one of the result information, the use information, the result of determination and the remaining life is written into the IC tag through the communication unit.

8. The method of any of claims 1 to 4, wherein the inspection device includes a battery and is portable.

9. The method of any of claims 1 to 8, wherein the inspection device has a network function, and is capable of transmitting at least one of the identification information, the use information, the remaining life and the result of determination to a separate machine element control database for storage therein.

10. The method of any of claims 1 to 9, wherein the inspection device has a network function, and is configured to read the identification information at the communication unit, transmit the identification information thus read to a separate check item control database, and receive, and temporarily store in the memory, the check item corresponding to the identification information, and the standard of determination or the determining equation corresponding to the check item.

11. The method of any of claims 1 to 10, wherein the machine element comprises a plurality of component parts, wherein the at least one IC tag comprises a plurality of IC tags mounted to the respective component parts of the machine element, and wherein the control unit determines whether or not the machine element is reusable based on whether or not all of the component parts of the machine element satisfy the standard of determination, or based on the result of calculation using the determining equation corresponding to the check item for all of the component parts of the machine element.

12. The method of claim 11, wherein the machine element is a bearing comprising a plurality of component parts, and wherein the at least one IC tag comprises a plurality of IC tags mounted to the respective component parts of the machine element.

13. The method of claim 11 or 12, wherein the machine element is disassembled into the individual component parts before reading information stored in the respective IC tags.

14. The method of any of claims 1 to 13, wherein the machine element includes a portion made of a metal, and wherein the IC tag is of a type into which information can be written and from which information can be read even if the IC tag is attached to or embedded in the portion made of a metal.

15. The method of any of claims 1 to 14, wherein determination can be made for the at least one check item or all of a plurality of check items without using a special-purpose measuring device.

# Fig.1

# Fig.2

IC tag 11

Control unit 43

Communication unit 41

Memory 42

Use information 55

Identification information 50

Result of determination 54a

Result information 53

Remaining life 54b

Communication unit 32

Memory 33

Identification information 50

Check items 51
Standard of determination 52a
Determining equation 52b

Standard of history determination 56

Network functional unit 37

Display 34

Control unit 31
(Determination unit 36)

Input unit 35

Battery 39

Inspection device 30

Network 81

Machine element control DB 61

Check item control DB 71

## Fig.3

### (a)

Model No.-check items
correspondence table

| Model No. | Check item |
|---|---|
| TT-34 | A12, A14, A16, M1, M2 |
| TT-54 | A12, A14, A17, M1, M2 |
| TT-57M | A12, A14, A17, M1, M3, M4 |
| TT-78N | A12, A15, A21, M1, M3, M4 |
| . | . |
| . | . |
| . | . |
| WEM5454 | F15 |
| WEN3375 | F15, F16 |
| . | . |
| . | . |
| . | . |

### (c)

Entered use infomation table

| Item No. | Content | Standard of determination |
|---|---|---|
| M1 | Total use time (hours) | 12000> |
| M2 | Normal rotational speed (rpm) | EX |
| M3 | Load (N) | EX |
| M4 | Frequency (number of times) | EX |
| | | |
| | | |

### (b)

Check item table

| Item No. | Detailed content of check item | Standard of determination |
|---|---|---|
| A11 | Visually check if there is any crack in outer race. | No |
| A12 | Check if there is any peeling on raceway through magnifying lens. | No |
| A13 | Check if there is any crack in the retainer through a magnifying lens. | No |
| A14 | Visually check if there is any rust on raceway. | No |
| . | . | . |
| . | . | . |
| F15 | Shed polarized light on center of reflecting surface and measure phase difference. | >35 |
| F16 | Measure the intensity of sonic wave of 315 Hz by acoustic analysis. | 55<x<114 |
| . | . | . |
| . | . | . |
| . | . | . |
| . | . | . |

EP 2 690 516 A1

# Fig.4

# Fig.5

Start (S101)

Reading ID information 50 from IC tag 11 by inspection device 30 (S102)

Reading check items 51 and other information from memory 33 based on ID information 50 (S103)

Displaying use information 55 to be entered (S104)

Entering use information 55 through input unit 35 (S105)

N ← Form acceptable?

Y

Temporarily storing use information 55 entered in memory 33 (S107)

Use information still remaining? (S108) → Y

N

n = 1 (S111)

Displaying nth check item 51 (S112)

Entering result information 53 through input unit 35 (S113)

N ← Form acceptable?

Y

Temporarily storing result information 53 entered in memory 33 (S115)

n = number of check items? (S116) → Y

N

Adding one to n (S117)

Determining all of the temporary information in memory 33 (S121)

Displaying result of determination 54a on display 34 (S122)

Writing result of determination 54a and use information 55 in IC tag 12 (S123)

End (S131)

# Fig.6

Start (S151)

Reading ID information 50 from IC tag 11 by inspection device 30 (S152)

Reading check items 51 and other information from memory 33 based on ID information 50 (S153)

Displaying use information 55 to be entered (S154)

Entering use information 55 through input unit 35 (S155)

Form acceptable? — N

Y

Temporarily storing use information 55 entered in memory 33 (S157)

Use information still remaining? (S158) — Y

N

n = 1 (S161)

Displaying nth one of check items 51 to be measured (S162)

Entering result information 53 through input unit 35 (S163)

Form acceptable? — N

Temporarily storing result information 53 entered in memory 33 (S165)

n = number of check items? (S166) — Y

N

Adding one to n (S167)

Executing determining equation 52b based on temporary information in memory 33 (S171)

Remaining life calculated? (S172) — Y

N

Reading check items 51 and other information from memory 33 based on result of determination (S173)

Display remaining life 54b on display 34 (S181)

Writing remaining life 54b, use information, etc. in IC tag 12 (S182)

End (S183)

25

# Fig.7

EP 2 690 516 A1

# Fig.8

EP 2 690 516 A1

# Fig.9

(a)

(b)

(c)

# Fig.10

(a)

(b)

(c)

## Fig.11

## Fig.12

# Fig.13

# Fig.14

(a)

(b)

Fig.15

(a)

(b)

# Fig.16

(a)

(b)

(c)

(d)

(e)

# Fig.17

# Fig.18

(a)

(b)

## INTERNATIONAL SEARCH REPORT

| | International application No. |
|---|---|
| | PCT/JP2012/057012 |

**A. CLASSIFICATION OF SUBJECT MATTER**
*G05B19/418*(2006.01)i

According to International Patent Classification (IPC) or to both national classification and IPC

**B. FIELDS SEARCHED**

Minimum documentation searched (classification system followed by classification symbols)
G05B19/418

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2006-85573 A (Hitachi Plant Engineering & Construction Co., Ltd.), 30 March 2006 (30.03.2006), paragraphs [0016] to [0029]; fig. 1 to 3 (Family: none) | 1-15 |
| Y | JP 2006-48584 A (Aeon Techno Service Co., Ltd.), 16 February 2006 (16.02.2006), paragraphs [0012] to [0043]; fig. 1 to 5 (Family: none) | 1-15 |
| Y | JP 2006-31231 A (Toshiba Corp.), 02 February 2006 (02.02.2006), paragraph [0017] (Family: none) | 1-15 |

| ☒ Further documents are listed in the continuation of Box C. | ☐ See patent family annex. |
|---|---|

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 06 April, 2012 (06.04.12) | 17 April, 2012 (17.04.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
|---|
| PCT/JP2012/057012 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 59-170901 A  (Hitachi, Ltd.),<br>27 September 1984 (27.09.1984),<br>page 2, upper left column<br>(Family: none) | 11-15 |
| Y | JP 2005-84962 A  (NTN Corp.),<br>31 March 2005 (31.03.2005),<br>entire text; all drawings<br>& US 2006/0289620 A1    & WO 2005/024534 A1<br>& DE 112004001668 T     & CN 1849568 A | 12-15 |

Form PCT/ISA/210 (continuation of second sheet) (July 2009)

**EP 2 690 516 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006053670 A
- JP 2006226498 A
- JP 4504065 B